# EUROPÄISCHE PATENTSCHRIFT

(11) **EP 1 072 242 B1**
(45) Veröffentlichungstag und Bekanntmachung des Hinweises auf die Patenterteilung: **08.08.2007**
(21) Anmeldenummer: 00111736.5
(22) Anmeldetag: 02.06.2000
(51) Int. Cl.: A61F 13/02

(54) **Pflaster sowie Verfahren zum Herstellen eines Pflasters**
Plaster and method of production of the plaster
Pansement et procédé de fabrication de pansement

(30) Priorität: 23.07.1999 DE 19934591
(43) Veröffentlichungstag der Anmeldung: 31.01.2001
(73) Patentinhaber: Paul Hartmann Aktiengesellschaft, 89522 Heidenheim (DE)
(72) Erfinder: Plaatje, Eckhard, 89522 Heidenheim (DE); Hermann, Klaus, 89537 Giengen (DE)
(74) Vertreter: Dreiss, Fuhlendorf, Steimle & Becker

(56) Entgegenhaltungen:
- EP-A- 0 109 280
- WO-A-97/42922

## Beschreibung

Die Erfindung betrifft ein Pflaster sowie ein Verfahren zum Herstellen desselben, wobei das Pflaster einen porösen, sich in einer Längsrichtung erstreckenden Träger umfasst, wobei eine Seite des Trägers wenigstens abschnittsweise mit einem selbstklebenden Schmelzkleber beschichtet ist.

Derzeit am Markt erhältliche Pflaster (mit Lösemittelkleber) werden zum einen als Rollen konfektioniert, wobei der Kleber im aufgerollten Zustand mit der Pflasteroberseite der darunter liegenden Wicklung in Kontakt ist, und zum anderen in Form von herkömmlichen Pflasterstreifen, bei denen zum Schutz der Klebeoberfläche auf dieser Schutzstreifen aus einem Kunststoffmaterial angeordnet sind, die vor Verwendung abgezogen werden.

Pflaster der gattungsgemäßen Art mit Schmelzkleberauftrag sind z. B. aus der EP 0 689 408 B1 und der EP 0 826 380 A2 bekannt. Da der Schmelzkleber selbst nicht atmungsaktiv ist, wird in der Regel kein vollflächiger Kleberauftrag vorgesehen, sondern der Schmelzkleberauftrag erfolgt bereichsweise. Der Auftrag kann z. B. streifenförmig in Längsrichtung des Pflasters, aber auch in Form von Querstreifen, punktförmig, rasterförmig oder in Form eines porösen Auftrags erfolgen, so dass die Atmungsaktivität sichergestellt ist.

Es erweist sich bei den bekannten Pflastern als problematisch, dass das Trägermaterial an seinen Randbereichen zum Ausfransen oder Ausfasern neigt, wodurch das Pflaster zum einen unansehnlich wird, zum anderen aber in seiner Form verändert wird. Des weiteren besteht das Problem, daß insbesondere beim Abziehen des Pflasters von einer Rolle, oder allgemein beim Ausfransen oder Ausfasern von Randbereichen Staub entsteht, was die Handhabung und Verwendung der Pflaster im medizinischen Bereich in der Nähe von offenen Wunden problematisch erscheinen läßt.

Aufgabe der Erfindung ist es daher, ein Pflaster sowie ein Verfahren zum Herstellen eines Pflasters bereitzustellen, bei dem das vorstehende Problem nicht oder in geringerem Maße auftritt.

Die Erfindung löst diese Aufgabe durch ein Pflaster, bei dem der Schmelzkleber entlang wenigstens eines Randbereichs des Trägers weiter in den porösen Träger eingedrungen ist als in zu dem Randbereich benachbarten Bereichen.

Das heißt, dass in den Randbereichen des Trägers bzw. des Pflasters der Schmelzkleber weiter in das Trägermaterial, also in die Poren des Trägers eingedrungen ist, als dies in den übrigen, lediglich mit dem Schmelzkleber beschichteten Bereichen der Fall ist.

Nach einer weiteren Variante der Erfindung ist der Schmelzkleber entlang wenigstens eines Randbereiches des Trägers in den porösen Träger wenigstens über 70 % der Dicke D des Trägers in diesen eingedrungen. Nach dieser zweiten Variante ist also vorgesehen, dass der Schmelzkleber wenigstens entlang eines Randbereichs, durchaus aber auch über die gesamte Erstreckung des Trägers in diesen eingedrungen ist. Auch hierdurch lässt sich eine Stabilisierung der Randbereiche erreichen.

Durch das Eindringen des Schmelzklebers in den porösen Träger werden z. B. die Fasern eines textilen Gewebes, aus dem der poröse Träger bestehen kann, miteinander verklebt. D. h. die Fäden, Kette und Schuss, werden untereinander durch den Schmelzkleber, der selbstklebend ist, verbunden, so dass kein Ausfransen bzw. Aufribbeln des Gewebes an den Randbereichen des Pflasters erfolgt. Mit dem Eindringen des Schmelzklebers in den porösen Träger in Randbereichen ist noch ein weiterer Vorteil verbunden, und zwar wird hierdurch eine Art Anfaßbereich ("Fingerlift") gebildet, weil der zur Haftvermittlung mit der Haut zur Verfügung stehende Schmelzkleber infolge des Eindringens in den porösen Träger reduziert ist. Daher ist auch die Haftkraft in diesem Randbereich reduziert, und ein Pflaster läßt sich beispielsweise problemlos an seinem Randbereich mit den Fingern ergreifen, um es von der Hautoberfläche abziehen zu können.

Bereiche, in denen der Schmelzkleber weiter in den porösen Träger eingedrungen ist als in hierzu benachbarten Bereichen, können zusätzlich auch z. B. in der Mitte des Pflasters vorgesehen sein. Hierdurch kann insgesamt eine höhere Beständigkeit des Pflasters erreicht werden.

Die Randbereiche können sich sowohl in Längsrichtung als auch in Querrichtung erstrecken. So werden bei Fixierpflastern im Wesentlichen die Längsrandbereiche so ausgebildet sein, dass der Schmelzkleber weiter in den Träger eingedrungen ist als in den benachbarten Bereichen. Bei herkömmlichen Heftpflasterstreifen kann dies jedoch sowohl an den Längs- als auch an den Querrandbereichen vorgesehen sein.

Der poröse Träger kann bspw. aus Vliesmaterial oder textilem Material, beispielsweise Gewebe, hergestellt sein.

Nach einem Ausführungsbeispiel erstreckt sich der Schmelzkleber im Randbereich wenigstens über 40 % der Dicke des Trägers in diesen hinein. Hierdurch wird bereits eine im Wesentlichen ausreichende Fixierung der Bestandteile des Trägers untereinander erreicht. Insbesondere kann vorgesehen sein, dass er sich über 50 % der Dicke des Trägers in diesen hineinerstreckt. Weiter kann vorgesehen sein, dass er sich insbesondere über 60 % der Dicke des Trägers in den Träger im Randbereich hineinerstreckt und schließlich kann insbesondere vorgesehen sein, dass er sich wenigstens über 70 % der Dicke in diesen hineinerstreckt. Hierdurch soll auch bei Textilien und Vliesmaterialien sichergestellt werden, dass ein Auflösen der Randbereiche vermieden wird, indem im Wesentlichen alle Fasern vom Schmelzkleber erfasst werden. Die Stabilität gegen Ausfransen bzw. Ausflusen der Randbereiche wird umso mehr gesteigert, je tiefer der Schmelzkleber im Randbereich in dem Querschnitt des Trägers vorliegt.

Das Pflaster kann bspw. so ausgebildet sein, dass der Schmelzkleber in in Längsrichtung verlaufenden Streifen auf dem Träger angebracht ist, wobei ein Schmelzkleberstreifen entlang wenigstens eines sich in Längsrichtung erstreckenden Randbereiches angeordnet ist. Durch eine derartige Anordnung wird die Atmungsaktivität des Pflasters sichergestellt, da zwischen den mit Schmelzkleber versehenen Bereichen Bereiche vorgesehen sind, die nicht beschichtet sind und bei denen die Luft so durch das poröse Trägermaterial hindurch zur Haut gelangen kann. Darüber hinaus bietet der in Längsstreifen aufgebrachte Schmelzkleber den Vorteil, dass der gesamte Randbereich durch einen einzigen Schmelzkleberstreifen gegen Ausfransen gesichert werden kann.

Es kann vorgesehen sein, sofern das Pflaster als herkömmliches "Heftpflaster" ausgebildet ist, dass auf der mit dem Schmelzkleber versehenen Seite des porösen Trägers ein Wundkissen zwischen den sich in Längs- und/oder Querrichtung erstreckenden Randbereichen angeordnet ist. Derartige Wundkissen dienen dazu, kleinere Wunden abzudecken und gleichzeitig das Wundkissen durch das poröse Trägermaterial, das mit dem Schmelzkleber beschichtet ist, sicher auf der Wunde zu fixieren. Das gleiche wird bei größeren Verletzungen durch die Verwendung von Wundabdeckungen in Verbindung mit Fixierpflastern, die ringsum um die Wundabdeckung angebracht werden, sichergestellt.

Um dem Ausfransen der Randbereiche weiter entgegenzuwirken, kann vorgesehen sein, dass die sich in Längs- und/oder Querrichtung erstreckenden Randbereiche des Trägers einen Wellen- oder Zickzackschnitt aufweisen.

Darüber hinaus wird ein Verfahren zum Herstellen eines Pflasters vorgeschlagen, wobei auf einer Seite einer sich in Längsrichtung erstreckenden aus einem porösen Trägermaterial gebildeten Flachmaterialbahn diskontinuierlich ein selbstklebender Schmelzkleber aufgebracht wird und die Flachmaterialbahn dann in die einzelnen Pflaster in Längs- und/oder Querrichtung geschnitten wird, wobei der aufgebrachte Schmelzkleber vor dem Schneiden zumindest entlang eines späteren Randbereiches des Pflasters erwärmt und in dem wenigstens einen Randbereich in den porösen Träger hineinverdrängt wird, so dass er in die Poren des porösen Trägermaterials eindringt. Das heißt, die aus dem porösen Trägermaterial gebildete Flachmaterialbahn wird zunächst in üblicher Weise diskontinuierlich mit dem selbstklebenden Schmelzkleber beschichtet, woraufhin dann der Schmelzkleber entlang der späteren Randbereiche erhitzt und in die Poren des porösen Trägermaterials hineinverdrängt wird. Erhitzen und Verdrängen des Schmelzklebers kann beispielsweise durch direkten Kontakt mit einem Heizkopf erreicht werden. Die Erhitzung kann aber auch in anderer Weise erfolgen, beispielsweise Heißluft, UV, IR, etc. Anschließend wird die Flachmaterialbahn dann entlang dieser Randbereiche geschnitten, und zwar vorzugsweise derart, dass der Schnitt durch die so behandelte Spur verläuft, so dass in den hierbei gebildeten Randbereichen der aus der Flachmaterialbahn geschnittenen Pflaster der Schmelzkleber weiter in das poröse Trägermaterial eingedrungen ist als in hierzu benachbarten Bereichen.

Mit der weiteren Ausbildung des erfindungsgemäßen Verfahrens nach Anspruch 14 wird sichergestellt, dass bei streifenförmigem Kleberauftrag stets mindestens ein Schmelzkleberstreifen von der Wärmeeinwirkung erfasst, erschmolzen und in das poröse Trägermaterial hineinverdrängt wird, um eine erfindungsgemäss herbeigeführte Spur zu bilden, durch die dann der Trennschnitt geführt werden kann, ohne dass besonders aufwendige Massnahmen zum Positionieren der Einrichtung zum Erhitzen und Erschmelzen des Klebers getroffen werden müssen.

Weitere Merkmale der Erfindung ergeben sich aus den übrigen Unterlagen.

Im Folgenden soll anhand einer Zeichnung ein bevorzugtes Ausführungsbeispiel näher erläutert werden.

Dabei zeigen:
- Fig. 1: eine perspektivische Darstellung eines handelsfertig konfektionierten Fixierpflasters;
- Fig. 2: in schematisierter Form den Herstellungsablauf eines derartigen Pflasters;
- Fig. 3: in schematisierter Form den Herstellungablauf eines derartigen Pflasters noch einer weiteren Variante.

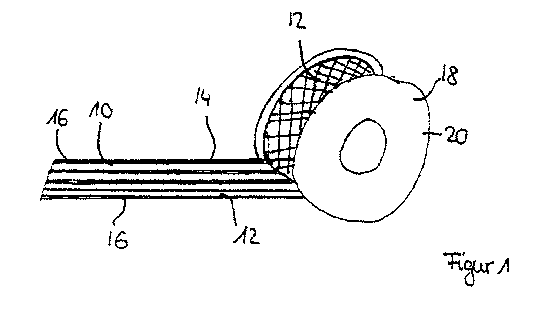

Fig. 1 zeigt dabei ein Pflaster 10, bestehend aus einem sich in Längsrichtung erstreckenden porösen Träger 12, der auf einer Seite diskontinuierlich, nämlich in Längsstreifen mit einem selbstklebenden Schmelzkleber 14 beschichtet ist. Der Schmelzkleber 14 ist dabei im Bereich beider Ränder des Trägers 12 aufgebracht. Darüber hinaus besteht auch ein Schmelzkleberauftrag in zu diesen Randbereichen 16 benachbarten Bereichen 17, die in der Mitte zwischen den Randbereichen 16 auf dem Träger 12 angeordnet sind. Das Pflaster 10 ist hierbei auf eine Pflasterrolle 18 aufgewickelt, die aus einer Hülse besteht, auf die das Pflaster 10 aufgewickelt ist, und aus zwei seitlichen Flanschen 20, die das Pflaster 10 zur Seite hin begrenzen. Das Trägermaterial besteht hierbei aus einem textilen Stoff, der eine gewisse Porosität aufweist und dadurch eine Atmungsaktivität des Pflasters 10 gewährleistet.

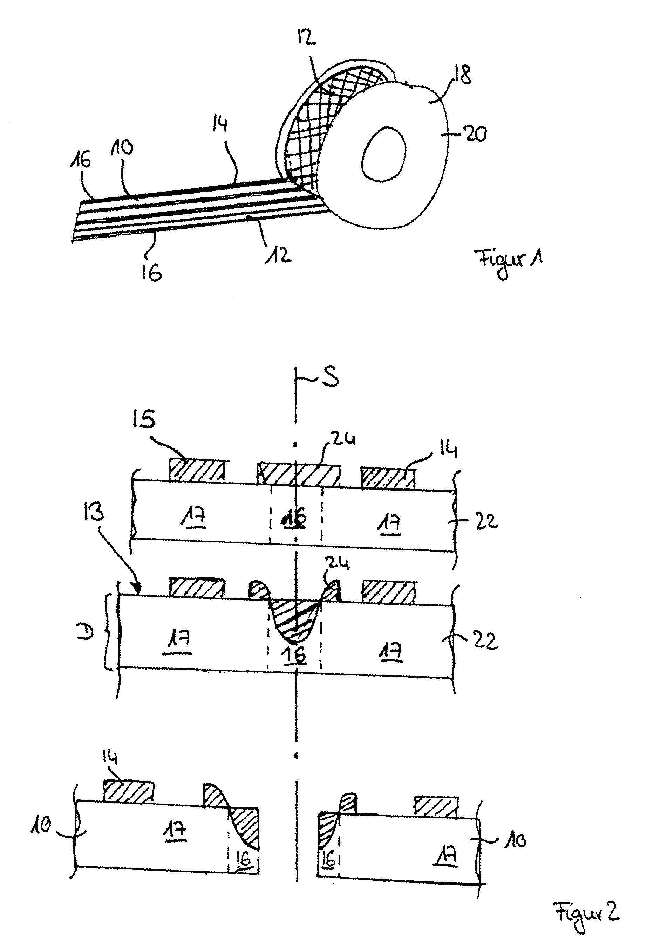

Fig. 2 zeigt ein Herstellungsverfahren zum Herstellen eines derartigen Pflasters 10. Hierbei ist auf einer Seite 13 einer sich in Längsrichtung erstreckenden teilweise gezeigten Flachmaterialbahn 22, die aus einem porösen Material, hier einem textilen Zellwollgewebe, besteht, diskontinuierlich, nämlich in Längsstreifen 15, ein Schmelzkleber 14 aufgebracht. Durch den diskontinuierlichen Auftrag wird die Atmungsaktivität des Pflasters 10 gewährleistet, da der Schmelzkleber 14 selbst nicht atmungsaktiv ist. Die Breite der Schmelzkleberstreifen 24 beträgt ca. 1 mm. Die Abstände zwischen den Schmelzkleberstreifen betragen etwa 0,5 mm. Das Flächengewicht des Schmelzkleberauftrags (Kleber + kleberfreie Bereiche) beträgt ca. 80 g/m². Als Schmelzklebermaterialien eignen sich die in den eingangs beschriebenen Patentveröffentlichungen erwähnten Kleber.

Die Flachmaterialbahn 22 ist von einem textilen Zellwollgewebe (Viskose) von ca. 115 g/m² gebildet. Die Fadendichte des Zellwollgewebes beträgt 33 Kettfäden pro Zentimeter und 29 Schußfäden pro Zentimeter.

Der Schmelzkleber 14 ist auf die Flachmaterialbahn 22 so aufgebracht, dass ein Schmelzkleberstreifen 24 im Bereich der späteren Längsrandbereiche 16 des Pflasters 10 angeordnet ist. Wie im zweiten Schritt dargestellt wurde, wird der Schmelzkleberstreifen 24 im Bereich der späteren Längsrandbereiche 16 erwärmt und durch einen Heizkopf (nicht dargestellt) zumindest teilweise in den porösen Träger 22 hineinverdrängt. Der Schmelzkleber 14 wird dabei verflüssigt und durch den über die Flachmaterialbahn 22 wandernden oder streichenden Heizkopf in die Poren des Trägermaterials eingebracht. Im dargestellten Fall wurde der Schmelzkleber derart in das Trägermaterial 22 hineinverdrängt, dass er sich über ca. 50 % der Dicke D bzw. des Querschnitts des Trägers 22 in diesen hineinerstreckt.

Anschließend wird, wie im dritten Bild zu erkennen ist, die Flachmaterialbahn 22 entlang der Linie S in Längsrichtung zerschnitten, so dass einzelne Fixierpflasterstreifen 10 entstehen. Der Schnitt erfolgt dabei mittig durch den Schmelzkleberstreifen 24.

Die nur teilweise gezeigten Pflaster 10 weisen dabei in ihren Längsrandbereichen 16 einen Schmelzkleberauftrag auf, der sich weiter in den porösen Träger hineinerstreckt als der Schmelzkleber 14 in den zu den Randbereichen 16 benachbarten Bereichen 17. Die einzelnen Pflaster 10 werden dann schließlich auf die in Figur 1 gezeigten Pflasterrollen 18 aufgerollt.

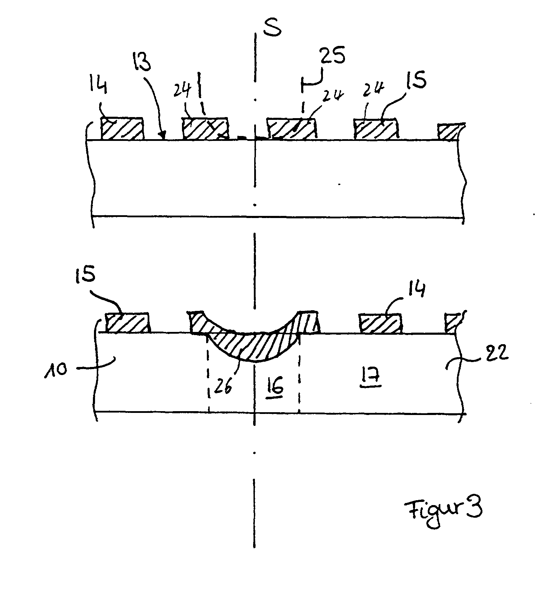

Figur 3 verdeutlicht eine alternative Variante zum Herstellen eines Pflasters 10, wobei wiederum von einem in Längsstreifen 15 auf eine Seite 13 einer Flachmaterialbahn 22 aufgebrachten Schmelzkleber 14 ausgegangen wird. Mit dem Bezugszeichen 25 ist schematisch ein Heizkopf dargestellt, dessen mit der Flachmaterialbahn 22 zusammenwirkende Kontaktfläche quer zur Längsrichtung der Streifen derart bemessen ist, daß sie den Zwischenraum zwischen zwei benachbarten Streifen 15 überbrückt und so zwei benachbarte Schmelzkleberstreifen 24 erfasst. Hierdurch werden die benachbarten Schmelzkleberstreifen 24 erschmolzen, wobei der Schmelzkleber in den zuvor kleberfreien Zwischenraum fliesst und dort unter der Wirkung des Heizkopfs 25 in den Träger 22 hineinverdrängt wird. Es entsteht eine im wesentlichen durch die Breite des Heizkopfs vorgegebene Kleberspur 26. Um sicherzustellen, dass der Heizkopf 25 oder eine an sich beliebige Einrichtung zum Erschmelzen des Schmelzklebers auch bei einem Bahnverlauf, d. h. bei einer unbeabsichtigen relativen Verlagerung des Trägers 22 zur Heiz- und Schneidvorrichtung quer zur Transportrichtung des Trägers einen Schmelzkleberstreifen 24 erfasst, ist dessen bzw. deren Breite wenigstens so breit wie die Summe der Breite eines Schmelzkleberstreifens 24 und der Breite des daran angrenzenden kleberfreien Bereichs.

## Patentansprüche

1. Pflaster umfassend einen porösen, sich in einer Längsrichtung erstreckenden Träger (12), wobei eine Seite (13) des Trägers (12) wenigstens abschnittsweise mit einem selbstklebenden Schmelzkleber (14) beschichtet ist, **dadurch gekennzeichnet, dass** der Schmelzkleber (14) entlang wenigstens eines Randbereiches (16) des Trägers (12) weiter in den porösen Träger (12) eingedrungen ist als in zu dem Randbereich (16) benachbarten Bereichen (17).

2. Pflaster umfassend einen porösen, sich in einer Längsrichtung erstreckenden Träger (12), wobei eine Seite (13) des Trägers (12) wenigstens abschnittsweise mit einem selbstklebenden Schmelzkleber (14) beschichtet ist, **dadurch gekennzeichnet, dass** der Schmelzkleber entlang wenigstens eines Randbereichs des Trägers in den porösen Träger (12) wenigstens über 70 % der Dicke (D) des Trägers (12) in diesen eingedrungen ist.

3. Pflaster nach Anspruch 1 oder 2, **dadurch gekennzeichnet, dass** der Randbereich (16) in Längsrichtung erstreckt ist.

4. Pflaster nach Anspruch 1, 2 oder 3, **dadurch gekennzeichnet, dass** der Randbereich (16) in Querrichtung erstreckt ist.

5. Pflaster nach Anspruch 1, 3 oder 4, **dadurch gekennzeichnet, dass** sich der Schmelzkleber (14) im Randbereich (16) wenigstens über 40% der Dicke (D) des Trägers (12) in diesen hineinerstreckt.

6. Pflaster nach Anspruch 5, **dadurch gekennzeichnet, dass** sich der Schmelzkleber (14) im Randbereich (16) wenigstens über 50% der Dicke (D) des Trägers (12) in diesen hineinerstreckt.

7. Pflaster nach Anspruch 6, **dadurch gekennzeichnet, dass** sich der Schmelzkleber (14) im Randbereich (16) wenigstens über 60% der Dicke (D) des Trägers (12) in diesen hineinerstreckt.

8. Pflaster nach einem Anspruch 7, **dadurch gekennzeichnet, dass** sich der Schmelzkleber (14) im Randbereich (16) wenigstens über 70% der Dicke (D) des Trägers (12) in diesen hineinerstreckt.

9. Pflaster nach einem der vorangehenden Ansprüche, **dadurch gekennzeichnet, dass** der poröse Träger (12) aus Vlies oder textilem Material besteht.

10. Pflaster nach einem der vorangehenden Ansprüche, **dadurch gekennzeichnet, dass** der Schmelzkleber (14) in in Längsrichtung verlaufenden Streifen (15) auf den Träger (12) aufgebracht ist, wobei ein Schmelzkleberstreifen (24) entlang wenigstens eines sich in Längsrichtung erstreckenden Randbereiches (16) angeordnet ist.

11. Pflaster nach einem der vorangehenden Ansprüche **dadurch gekennzeichnet, dass** auf der mit dem Schmelzkleber (14) versehenen Seite (13) des porösen Trägers (12) ein Wundkissen zwischen den sich in Längs- und/oder Querrichtung erstreckenden Randbereichen (16) angeordnet ist.

12. Pflaster nach einem der vorangehenden Ansprüche **dadurch gekennzeichnet, dass** die sich in Längs- und/oder Querrichtung erstreckenden Randbereiche (16) des Trägers (12) einen Wellen- oder Zick-Zack-Schnitt aufweisen.

13. Verfahren zum Herstellen eines Pflasters nach einem der Ansprüche 1 bis 12, wobei auf einer Seite (13) einer sich in eine Längsrichtung erstreckenden aus einem porösen Tägermaterial gebildeten Flachmaterialbahn (22) diskontinuierlich ein selbstklebender Schmelzkleber (14) aufgebracht wird und die Flachmaterialbahn (22) dann in die einzelnen Pflaster (10) in Längs- und/oder Querrichtung geschnitten wird, **dadurch gekennzeichnet, dass** der aufgebrachte Schmelzkleber (14) vor dem Schneiden zumindest entlang eines späteren Randbereiches (16) des Pflasters (10) erwärmt und in dem wenigstens einen Randbereich (16) in das poröse Trägermaterial hineinverdrängt wird, so dass er in die Poren des porösen Trägermaterials eindringt, wobei der Schmelzkleber (14) entlang wenigstens eines Randbereichs (16) des Trägers (12) weiter in den porösen Träger (12) hineinverdrängt wird als in zu dem Randbereich (16) benachbarten Bereichen (17) oder der Schmelzkleber entlang wenigstens eines Randbereichs des Trägers in den porösen Träger (12) wenigstens über 70 % der Dicke (D) des Trägers (12) in diesen hineinverdrängt wird.

14. Verfahren nach Anspruch 13, **dadurch gekennzeichnet, dass** zum Erschmelzen eines in Streifen (15, 24) aufgebrachten Schmelzklebers (14) die Flachmaterialbahn (22) über eine Breite quer zur Längsrichtung der Schmelzkleberstreifen (15, 24) erhitzt wird, die wenigstens gleich der Summe eines Schmelzkleberstreifens (15, 24) und der Breite des zwischen zwei Schmelzkleberstreifen (15, 24) befindlichen kleberfreien Bereichs bemessen ist.

## Claims

1. Plaster comprising a porous carrier (12) which extends in a longitudinal direction, one side (13) of the carrier (12) being coated, at least in portions, with a self-adhesive hot-melt adhesive (14), **characterised in that**, along at least one edge region (16) of the carrier (12), the hot-melt adhesive (14) penetrates further into the porous carrier (12) than in regions (17) adjacent to the edge region (16).

2. Plaster comprising a porous carrier (12) which extends in a longitudinal direction, one side (13) of the carrier (12) being coated, at least in portions, with a self-adhesive hot-melt adhesive (14), **characterised in that** the hot-melt adhesive penetrates into the porous carrier (12) along at least one edge region of the carrier (12) at least over 70 % of the thickness (D) thereof.

3. Plaster according to either claim 1 or claim 2, **characterised in that** the edge region (16) extends longitudinally.

4. Plaster according to claim 1, claim 2 or claim 3, **characterised in that** the edge region (16) extends transversely.

5. Plaster according to claim 1, claim 3 or claim 4, **characterised in that**, in the edge region (16), the holt-melt adhesive (14) extends into the carrier (12) at least over 40 % of the thickness (D) thereof.

6. Plaster according to claim 5, **characterised in that**, in the edge region (16), the hot-melt adhesive (14) extends into the carrier (12) at least over 50 % of the thickness (D) thereof.

7. Plaster according to claim 6, **characterised in that**, in the edge region (16), the hot-melt adhesive (14) extends into the carrier (12) at least over 60 % of the thickness (D) thereof.

8. Plaster according to claim 7, **characterised in that**, in the edge region (16), the hot-melt adhesive (14) extends into the carrier (12) at least over 70 % of the thickness (D) thereof.

9. Plaster according to any one of the preceding claims, **characterised in that** the porous carrier (12) is made of non-woven fabric or textile material.

10. Plaster according to any one of the preceding claims, **characterised in that** the hot-melt adhesive (14) is applied to the carrier (12) in strips (15) which extend longitudinally, a strip (24) of hot-melt adhesive being arranged along at least one edge region (16) which extends longitudinally.

11. Plaster according to any one of the preceding claims, **characterised in that** on the side (13) of the porous carrier (12) provided with the hot-melt adhesive (14), a wound pad is arranged between the edge regions (16) which extend longitudinally and/or transversely.

12. Plaster according to any one of the preceding claims, **characterised in that** the edge regions (16), which extend longitudinally and/or transversely, of the carrier (12) have a wavelike or zigzag cross-section.

13. Method for producing a plaster according to any one of claims 1 to 12, a self-adhesive hot-melt adhesive (14) being applied in a discontinuous manner on one side (13) of a flat material web (22), which extends in a longitudinal direction and is formed from a porous carrier material, and the flat material web (22) subsequently being cut longitudinally and/or transversely into the individual plasters (10), **characterised in that**, before cutting, the applied hot-melt adhesive (14) is heated at least along a subsequent edge region (16) of the plaster (10) and penetrates into the porous carrier material in the at least one edge region (16) so that it penetrates into the pores of the porous carrier material, the hot-melt adhesive (14) penetrating further into the porous carrier (12) along at least one edge region (16) of the carrier (12) than in regions (17) adjacent to the edge region (16), or the hot-melt adhesive penetrating into the porous carrier (12) along at least one edge region of the carrier (12) into at least over 70 % of the thickness (D) thereof.

14. Method according to claim 13, **characterised in that** in order to melt a hot-melt adhesive (14) applied in strips (15, 24), the flat material web (22) is heated over a width transverse to the longitudinal direction of the strips (15, 24) of hot-melt adhesive which is at least equal to the sum of the width of a strip (15, 24) of hot-melt adhesive and the width of the adhesive-free region located between two strips (15, 24) of hot-melt adhesive.

## Revendications

1. Pansement comportant un support poreux (12) s'étendant dans une direction longitudinale, un côté (13) du support (12) étant revêtu au moins par endroits d'une colle fusible auto-adhésive (14), **caractérisé en ce que** l'on fait pénétrer la colle fusible (14) plus profondément dans le support poreux (12) le long d'au moins une zone de bordure (16) du support (12) que dans des zones voisines (17) de la zone de bordure (16).

2. Pansement comportant un support poreux (12) s'étendant dans une direction longitudinale, un côté (13) du support (12) étant revêtu au moins par endroits d'une colle fusible auto-adhésive (14), **caractérisé en ce que**, le long d'au moins une zone de bordure du support, l'on fait pénétrer la colle fusible dans le support poreux (12) au moins sur 70 % de l'épaisseur (D) du support (12) à l'intérieur de celui-ci.

3. Pansement selon la revendication 1 ou 2, **caractérisé en ce que** la zone de bordure (16) s'étend dans le sens longitudinal.

4. Pansement selon la revendication 1, 2 ou 3, **caractérisé en ce que** la zone de bordure (16) s'étend dans le sens transversal.

5. Pansement selon la revendication 1, 3 ou 4, **caractérisé en ce que** la colle fusible (14) s'étend, dans la zone de bordure (16), au moins sur 40 % de l'épaisseur (D) du support (12) à l'intérieur de celui-ci.

6. Pansement selon la revendication 5, **caractérisé en ce que** la colle fusible (14) s'étend, dans la zone de bordure (16), au moins sur 50 % de l'épaisseur (D) du support (12) à l'intérieur de celui-ci.

7. Pansement selon la revendication 6, **caractérisé en ce que** la colle fusible (14) s'étend, dans la zone de bordure (16), au moins sur 60 % de l'épaisseur (D) du support (12) à l'intérieur de celui-ci.

8. Pansement selon la revendication 7, **caractérisé en ce que** la colle fusible (14) s'étend, dans la zone de bordure (16), au moins sur 70 % de l'épaisseur (D) du support (12) à l'intérieur de celui-ci.

9. Pansement selon l'une des revendications précédentes, **caractérisé en ce que** le support poreux (12) est en non-tissé ou en matière textile.

10. Pansement selon l'une des revendications précédentes, **caractérisé en ce que** la colle fusible (14) est appliquée sur le support (12) en bandes (15) s'étendant dans le sens longitudinal, une bande de colle fusible (24) étant agencée le long d'au moins une zone de bordure (16) s'étendant dans le sens longitudinal.

11. Pansement selon l'une des revendications précédentes, **caractérisé en ce que**, sur le côté (13) du support poreux (12) pourvu de la colle fusible (14), il est agencé une compresse centrale entre les zones de bordure (16) s'étendant dans le sens longitudinal et/ou transversal.

12. Pansement selon l'une des revendications précédentes, **caractérisé en ce que** les zones de bordure (16) du support (12) s'étendant dans le sens longitudinal et/ou transversal présentent une coupe ondulée ou en zigzag.

13. Procédé de fabrication d'un pansement selon l'une des revendications 1 à 12, une colle fusible auto-adhésive (14) étant appliquée de façon discontinue sur un côté (13) d'une bande de matière plate (22) formée par une matière de support poreuse s'étendant dans une direction longitudinale et la bande de matière plate (22) étant ensuite découpée dans le sens longitudinal et/ou transversal en pansements individuels (10), **caractérisé en ce que** la colle fusible (14) appliquée est chauffée avant le découpage au moins le long d'une zone de bordure ultérieure (16) du pansement (10) et poussée, dans au moins une zone de bordure (16), à l'intérieur de la matière de support poreuse, de telle sorte qu'elle pénètre dans les pores de la matière de support poreuse, la colle fusible (14) étant poussée plus profondément à l'intérieur du support poreux (12) le long d'au moins une zone de bordure (16) du support (12) que dans des zones voisines (17) de la zone de bordure (16) ou la colle fusible étant poussée le long d'au moins une zone de bordure du support dans le support poreux (12) au moins sur 70 % de l'épaisseur (D) du support (12) à l'intérieur de celui-ci.

14. Procédé selon la revendication 13, **caractérisé en ce que**, pour élaborer une colle fusible (14) appliquée en bandes (15, 24), la bande de matière plate (22) est chauffée sur une largeur transversale au sens longitudinal des bandes de colle fusible (15, 24), laquelle largeur est au moins égale à la somme d'une bande de colle fusible (15, 24) et de la largeur de la zone sans colle située entre deux bandes de colle fusible (15, 24).
